# EUROPEAN PATENT APPLICATION

(11) **EP 3 805 386 A1**
(43) Date of publication of application: **14.04.2021**
(21) Application number: 19815297.7
(22) Date of filing: 07.06.2019
(51) Int. Cl.: C12N 15/09, C07K 19/00, C12N 9/16, C12N 15/113, C12N 15/55, C12N 15/62

(54) **MODIFIED CAS9 PROTEIN AND USE THEREOF**

(30) Priority: 08.06.2018 US 201862682244 P
(71) Applicant: Modalis Therapeutics Corporation, Tokyo, 103-0023 (JP)
(72) Inventor: YAMAGATA, Tetsuya, Cambridge, MA 02138 (US); QIN, Yuanbo, Cambridge, MA 02138 (US)
(74) Representative: Hoffmann Eitle
(86) International application number: PCT/JP2019/022795
(87) International publication number: WO 2019/235627

(57) **Abstract**

A protein having a binding ability to guide RNA and consisting of a sequence containing an amino acid sequence wherein a continuous deletion region is present between the 481-position and the 649-position in the amino acid sequence shown in SEQ ID NO: 2, the deletion region containing
(i) all or a part of L1 domain (481- to 519-positions), and
(ii) entire HNH domain (520- to 628-positions), and further optionally containing
(iii) all or a part of L2 domain (629- to 649-positions), wherein amino acids adjacent to each of the deletion region are linked by a linker consisting of 3 to 10 amino acid residues functions as a miniaturized dSaCas9 protein while maintaining DNA binding affinity. Use of the miniaturized dSaCas9 protein makes it possible to mount many genes into vectors.

## Description

### [Technical Field]

The present invention relates to a modified Cas9 protein that is miniaturized while maintaining a binding ability to guide RNA, and use thereof.

### [Background Art]

Clustered Regularly Interspaced Short Palindromic Repeats (CRISPR) are known to compose the adaptive immune system that provides acquired resistance against invasive foreign nucleic acids in bacteria and archaea together with Cas (CRISPR-associated) genes. CRISPR frequently originate from phage or plasmid DNA and are composed of 24 bp to 48 bp short, conserved repeat sequences having unique variable DNA sequences referred to as spacers of similar size inserted there between. In addition, a group of genes encoding the Cas protein family is present in the vicinity of the repeat and spacer sequences.

In the CRISPR/Cas system, foreign DNA is cleaved into fragments of about 30 bp by the Cas protein family and inserted into CRISPR. Cas1 and Cas2 proteins, which are among the Cas protein family, recognize a base sequence referred to as proto-spacer adjacent motif (PAM) of foreign DNA, cut the upstream, and insert same into the CRISPR sequence of the host, which creates immune memory of bacteria. RNA generated by transcription of a CRISPR sequence including immune memory (referred to as pre-crRNA) is paired with a partially complementary RNA (trans-activating crRNA: tracrRNA) and incorporated into Cas9 protein which is one of the Cas protein family. The pre-crRNA and tracrRNA incorporated into Cas9 are cleaved by RNaseIII to form small RNA fragments (CRISPR-RNAs: crRNAs) containing a foreign sequence (guide sequence), and a Cas9-crRNA-tracrRNA complex is thus formed. The Cas9-crRNA-tracrRNA complex binds to a foreign invasive DNA complementary to crRNA, and the Cas9 protein, which is an enzyme that cleaves the DNA (nuclease), cleaves the foreign invasive DNA, thereby suppressing and eliminating the function of the DNA that invaded from the outside.

In recent years, techniques for applying the CRISPR/Cas system to genome editing have been actively developed. crRNA and tracrRNA are fused, expressed as a tracrRNA-crRNA chimera (hereinafter to be referred to as guide RNA: gRNA), and utilized. Using this, nuclease (RNA-guided nuclease: RGN) is then recruited to cleave genomic DNA at the target site.

On the other hand, a system that can regulate the expression level of the target gene can be obtained by fusing a transcriptional regulator such as transcriptional activator (e.g., VP64, VP160 and the like), a transcriptional inhibitor (e.g., KRAB and the like), and the like with a variant (nuclease-null, dCas9) wherein the nuclease of the Cas9 protein in CRISPR/Cas9, which is one of the genome editing systems, is inactivated. For example, to further increase the efficiency of gene activation, it is fused with an activation factor (VP64-p65-Rta, VPR) in which three transcriptional activators are linked, and the fused dCas9 protein (dCas9-VPR; dCas9 fusion protein) strongly activates expression of the target gene without cleaving DNA.

Various variants of Cas9 protein have been created and reported for the purpose of alleviating PAM specificity, modifying (activating/inactivating) nuclease activity, and miniaturizing size (patent documents 1 - 3).

The Cas9 protein consists of two lobes; a REC lobe (REC: recognition) and a NUC lobe (NUC: nuclease). The REC lobe is composed of an α-helix rich in arginine residues, a REC1 domain and a REC2 domain, and the NUC lobe is composed of a RuvC domain, an HNH domain and a PI domain (PI: PAM interacting). The RuvC domain contains three motifs (RuvC-I to RuvC-III). As a miniaturized Cas9 protein, SaCas9 (i.e., mini-SaCas9) has been reported in which all or part of each functional domain is removed and linked with a linker. As the linker, GS-linker (GGGGSGGGG: SEQ ID NO: 10), R-linker (KRRRRHR: SEQ ID NO: 11) and GSK linker (GSK) are known (patent document 4, non-patent document 1).

### [Document List]

### [Patent documents]

patent document 1: WO 2016/141224A1
patent document 2: WO 2017/010543A1
patent document 3: WO 2018/074979A1
patent document 4: WO 2018/209712A1

### [non-patent document]

non-patent document 1: Dacheng Ma, et al., ACS Synth. Biol. 2018, 7, 978-985

### [Summary of Invention]

### [Technical Problem]

Expression of dCas9 fusion protein in vivo requires an expression vector. In gene therapy, adeno-associated virus vector (AAV) is mainly used since it is highly safe and highly efficient. The mountable size of AAV is about 4.4 kb, while the dCas9 protein already occupies about 4 kb, and the constitution of fusion protein is extremely limited for mounting on AAV.

Therefore, the present inventors aim to provide a further miniaturized dCas9 protein variant having DNA binding affinity substantially equivalent to that of a full-length protein.

### [Solution to Problem]

The present inventors took note of a nuclease-null variant (dSaCas9) of Cas9 derived from S. aureus (to be also referred to as SaCas9 in the present description) as a Cas9 protein, and have conducted intensive studies in an attempt to solve the above-mentioned problems. As a result, they have found a specific region that has little influence on the ability to bind to guide RNA even if deleted, and succeeded in producing a miniaturized dSaCas9 protein while maintaining or enhancing the DNA binding affinity by substituting the amino acid at a predetermined position with a specific amino acid, which resulted in the completion of the present invention.

Deletion and substitution are also collectively referred to as mutation.

In the present description, dSaCas9 protein before introduction of mutation is sometimes to be referred to as wild-type dSaCas9 (protein), and dSaCas9 protein after introduction of mutation is sometimes to be referred to as modified dSaCas9 variant (protein).

That is, the present invention provides the following.
[1] A protein having a binding ability to guide RNA and consisting of a sequence comprising an amino acid sequence wherein a continuous deletion region is present between the 481-position and the 649-position in the amino acid sequence shown in SEQ ID NO: 2, the deletion region comprising
   (i) all or a part of L1 domain (481- to 519-positions), and
   (ii) entire HNH domain (520- to 628-positions), and further optionally comprising
   (iii) all or a part of L2 domain (629- to 649-positions), wherein amino acids adjacent to each of the deletion region are linked by a linker consisting of 3 to 10 amino acid residues.
[2] The protein of the above-mentioned [1], wherein the deletion region comprises
   (i) entire L1 domain (481- to 519-positions),
   (ii) entire HNH domain region (520- to 628-positions), and
   (iii) entire L2 domain (629- to 649-positions).
[3] The protein of the above-mentioned [1], wherein the deletion region comprises
   (i) a part of L1 domain (482- to 519-positions),
   (ii) entire HNH domain (520- to 628-positions), and
   (iii) a part of L2 domain (629- to 647-positions).
[4] The protein of the above-mentioned [1], wherein the deletion region comprises
   (i) a part of L1 domain (482- to 519-positions), and
   (ii) entire HNH domain (520- to 628-positions).
[5] A protein consisting of a sequence comprising an amino acid sequence resulting from substitution of glutamic acid (E) at the 45-position and/or the 163-position with other amino acid in the amino acid sequence shown in SEQ ID NO: 2, and having a binding ability to guide RNA.
[6] The protein of the above-mentioned [5], wherein said other amino acid is a basic amino acid.
[7] The protein of the above-mentioned [6], wherein the basic amino acid is lysine (K).
[8] The protein of any of the above-mentioned [1] to [4], wherein glutamic acid (E) at the 45-position and/or the 163-position are/is substituted with other amino acid(s).
[9] The protein of the above-mentioned [8], wherein said other amino acid is a basic amino acid.
[10] The protein of the above-mentioned [9], wherein the basic amino acid is lysine (K).
[11] The protein of any of the above-mentioned [1] to [8], wherein the linker is a 5 - 9 amino acid length linker composed of glycine (G) and serine (S).
[12] The protein of any of the above-mentioned [1] to [11], wherein the linker is selected from the following:
   - SGGGS-
   - GGSGGS-
   - SGSGSGSG-
   - SGSGSGSGS-.
[13] The protein of any of the above-mentioned [1] to [12], having identity of 80% or more at a site other than the mutated and/or deleted positions in the SEQ ID NO: 2.
[14] The protein of any of the above-mentioned [1] to [12], wherein one to several amino acids are substituted, deleted, inserted and/or added at a site other than the mutated and/or deleted positions in the SEQ ID NO: 2.
[15] The protein of any of the above-mentioned [1] to [14], wherein a transcriptional regulator protein or domain is linked.
[16] The protein of the above-mentioned [15], wherein the transcriptional regulator is a transcriptional activator.
[17] The protein of the above-mentioned [15], wherein the transcriptional regulator is a transcriptional silencer or a transcriptional inhibitor.
[18] A nucleic acid encoding the protein of any of the above-mentioned [1] to [17].
[19] A protein-RNA complex provided with the protein of any of the above-mentioned [1] to [18] and a guide RNA comprising a polynucleotide composed of a base sequence complementary to a base sequence located 1 to 20 to 24 bases upstream from a proto-spacer adjacent motif (PAM) sequence in a target double-stranded polynucleotide.
[20] A method for site-specifically modifying a target double-stranded polynucleotide, including
   a step of mixing and incubating a target double-stranded polynucleotide, a protein and a guide RNA, and
   a step of having the aforementioned protein modify the aforementioned target double-stranded polynucleotide at a binding site located upstream of a PAM sequence; wherein,
   the aforementioned protein is the protein of any of the above-mentioned [1] to [17], and
   the aforementioned guide RNA contains a polynucleotide composed of a base sequence complementary to a base sequence located 1 to 20 to 24 bases upstream from the aforementioned PAM sequence in the aforementioned target double-stranded polynucleotide.
[21] A method for increasing expression of a target gene in a cell, comprising expressing the protein of the above-mentioned [16] and one or plural guide RNAs for the aforementioned target gene in the aforementioned cell.
[22] A method for decreasing expression of a target gene in a cell, comprising expressing the protein of the above-mentioned [17] and one or plural guide RNAs for the aforementioned target gene in the aforementioned cell.
[23] The method of the above-mentioned [21] or [22], wherein the cell is a eukaryotic cell.
[24] The method of the above-mentioned [21] or [22], wherein the cell is a yeast cell, a plant cell or an animal cell.

### [Advantageous Effects of Invention]

According to the present invention, a dSaCas9 protein further miniaturized while having a binding ability to guide RNA can be obtained. The miniaturized dSaCas9 protein makes it possible to mount a larger number of genes into expression vectors limited in capacity.

### [Brief Description of Drawings]

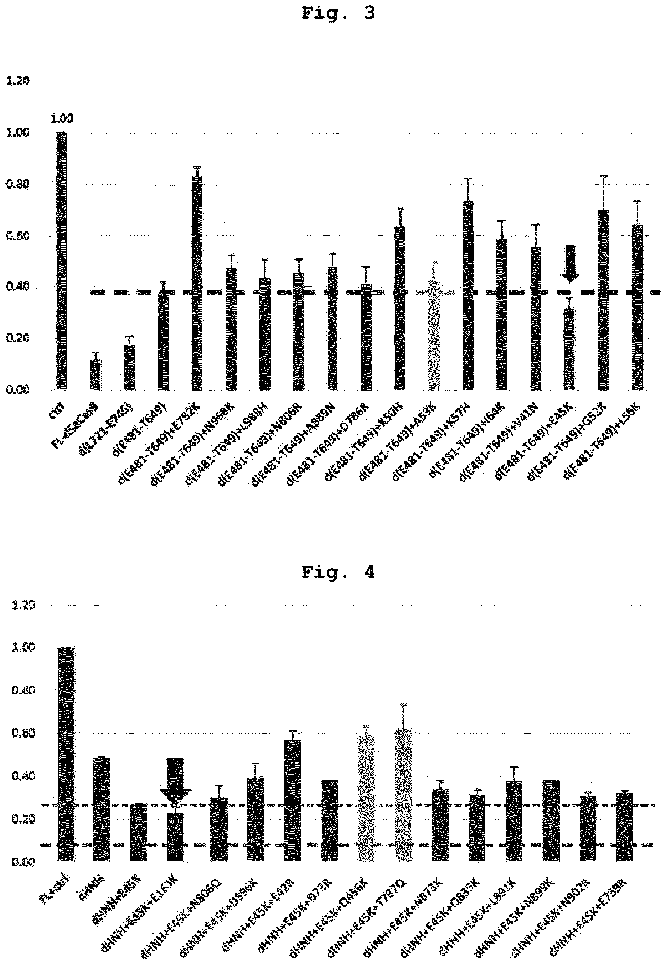

Fig. 1 is a schematic showing of the structure of wild-type dSaCas9(WT) and dSaCas9 variants (T1 - T3).
   T1: dsaCas9-d(E481-T649) with "GGSGGS" as linker,
   T2: dsaCas9-d(K482-V647) with "SGGGS" as linker,
   T3: dsaCas9-d(K482-E628) with "SGGGS" as linker
Fig. 2 is a graph showing the DNA binding affinity of wild-type dSaCas9(WT) and dSaCas9 variants (T1 - T3).
   T1: dsaCas9-d(E481-T649) with "GGSGGS" as linker,
   T2: dsaCas9-d(K482-V647) with "SGGGS" as linker,
   T3: dsaCas9-d(K482-E628) with "SGGGS" as linker
Fig. 3 is a graph showing the DNA binding affinity of wild-type dSaCas9(WT) and dSaCas9 variants (M1 - M14).
   M1: E782K on dsaCas9-d(E481-T649) with "GGSGGS" as linker,
   M2: N968K on dsaCas9-d(E481-T649) with "GGSGGS" as linker,
   M3: L988H on dsaCas9-d(E481-T649) with "GGSGGS" as linker,
   M4: N806R on dsaCas9-d(E481-T649) with "GGSGGS" as linker,
   M5: A889N on dsaCas9-d(E481-T649) with "GGSGGS" as linker,
   M6: D786R on dsaCas9-d(E481-T649) with "GGSGGS" as linker,
   M7: K50H on dsaCas9-d(E481-T649) with "GGSGGS" as linker,
   M8: A53K on dsaCas9-d(E481-T649) with "GGSGGS" as linker,
   M9: K57H on dsaCas9-d(E481-T649) with "GGSGGS" as linker,
   M10: I64K on dsaCas9-d(E481-T649) with "GGSGGS" as linker,
   M11: V41N on dsaCas9-d(E481-T649) with "GGSGGS" as linker,
   M12: E45K on dsaCas9-d(E481-T649) with "GGSGGS" as linker,
   M13: G52K on dsaCas9-d(E481-T649) with "GGSGGS" as linker,
   M14: L56K on dsaCas9-d(E481-T649) with "GGSGGS" as linker
Fig. 4 is a graph showing the DNA binding affinity of wild-type dSaCas9(WT) and dSaCas9 variants (M15 - M27).
   M15: E163K on dsaCas9-d(E481-T649) + E45K,
   M16: N806Q on dsaCas9-d(E481-T649) + E45K,
   M17: D896K on dsaCas9-d(E481-T649) + E45K,
   M18: E42R on dsaCas9-d(E481-T649) + E45K,
   M19: D73R on dsaCas9-d(E481-T649) + E45K,
   M20: Q456K on dsaCas9-d(E481-T649) + E45K,
   M21: T787Q on dsaCas9-d(E481-T649) + E45K,
   M22: N873K on dsaCas9-d(E481-T649) + E45K,
   M23: Q835K on dsaCas9-d(E481-T649) + E45K,
   M24: L891K on dsaCas9-d(E481-T649) + E45K,
   M25: N899K on dsaCas9-d(E481-T649) + E45K,
   M26: N902R on dsaCas9-d(E481-T649) + E45K,
   M27: E739R on dsaCas9-d(E481-T649) + E45K

### [Description of Embodiments]

The present invention is described below. Unless particularly indicated, the terms used in the present description have meanings generally used in the pertinent field.

### <dSaCas9 variant>

The dSaCas9 variant of the present invention is a dSaCas9 protein further miniaturized while having a binding ability to guide RNA. Using the miniaturized dSaCas9 protein, a larger number of genes can be mounted into a vector.

In the present description, "guide RNA" refers to that which mimics the hairpin structure of tracrRNA-crRNA, and contains in the 5'-terminal region thereof a polynucleotide composed of a base sequence complementary to a base sequence located from 1 to preferably 20 to 24 bases, and more preferably from 1 to preferably 22 to 24 bases, upstream from the PAM sequence in a target double-stranded polynucleotide. Moreover, guide RNA may contain one or more polynucleotides composed of a base sequence allowing the obtaining of a hairpin structure composed of base sequences non-complementary to a target double-stranded polynucleotide symmetrically arranged so as to form a complementary sequence having a single point as the axis thereof.

The guide RNA has a function of binding to the dSaCas9 variant of the present invention and leading the protein to a target DNA. The guide RNA has a sequence at the 5'-terminal which is complementary to the target DNA, and binds to the target DNA via the complementary sequence, thereby leading the dSaCas9 variant of the present invention to the target DNA. Since the dSaCas9 variant does not have a DNA endonuclease, it does not cleave target DNA though it binds to the target DNA.

The guide RNA is designed and prepared based on the sequence information of the target DNA. Specific examples include sequences such as those used in the Examples.

In the present description, the terms "polypeptide", "peptide" and "protein" refer to polymers of amino acid residues and are used interchangeably. In addition, these terms also refer to amino acid polymers in which one or a plurality of amino acid residues are in the form of a chemical analog or modified derivative of the corresponding amino acids present in nature.

In the present description, the "basic amino acid" refers to an amino acid having a residue showing basicity in addition to one amino group in a molecule such as lysine, arginine, histidine and the like.

In the present description, a "sequence" refers to a nucleotide sequence of an arbitrary length, is a deoxyribonucleotide or ribonucleotide, and may be linear or branched and single-stranded or double-stranded.

In the present description, a "PAM sequence" refers to a sequence present in a target double-stranded polynucleotide that can be recognized by Cas9 protein, and the length and base sequence of the PAM sequence differs according to the bacterial species.

Furthermore, in the present description, "N" refers to any one base selected from the group consisting of adenine, cytosine, thymine and guanine, "A" refers to adenine, "G" to guanine, "C" to cytosine, "T" to thymine, "R" to a base having a purine skeleton (adenine or guanine), and "Y" to a base having a pyrimidine skeleton (cytosine or thymine).

In the present description, a "polynucleotide" refers to a deoxyribonucleotide or ribonucleotide polymer having linear or cyclic coordination and may be single-stranded or double-stranded, and should not be interpreted as being restricted with respect to polymer length. In addition, polynucleotides include known analogs of naturally-occurring nucleotides as well as nucleotides in which at least one of the base moieties, sugar moieties and phosphate moieties thereof has been modified (such as a phosphorothioate backbone). In general, an analog of a specific nucleotide has the same base-pairing specificity, and for example, A analogs form base pairs with T.

The present invention provides a protein (embodiment 1) having a binding ability to guide RNA and consisting of a sequence comprising an amino acid sequence wherein a continuous deletion region is present between the 481-position and 649-positions in the amino acid sequence shown in SEQ ID NO: 2, the deletion region comprising
(i) all or a part of L1 domain (481- to 519-positions), and
(ii) entire HNH domain (520- to 628-positions), and further optionally containing
(iii) all or a part of L2 domain (629- to 649-positions), wherein amino acids adjacent to each of the deletion region are linked by a linker consisting of 3 to 10 amino acid residues.

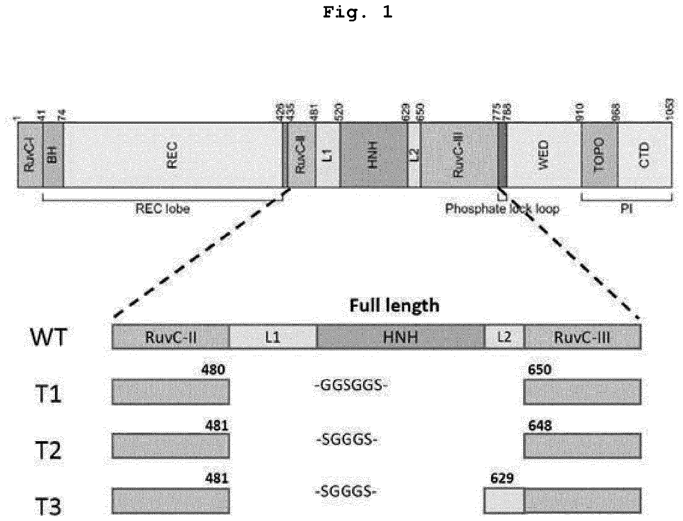

SEQ ID NO: 2 is a full-length amino acid sequence of dSaCas9 protein. The dSaCas9 protein is SaCas9 (S. aureus-derived Cas9) in which the 10-position aspartic acid is substituted with alanine, and the 580-position asparagine is substituted with alanine, and consists of two lobes of REC lobe (41 - 425 residues) and NUC lobe (1 - 40 residues and 435 - 1053 residues) as also shown in Fig. 1. The two lobes are linked via bridge helix (BH: 41 - 73 residues) rich in arginine and linker loop (426 - 434 residues). The NUC lobe is constituted of RuvC domain (1 - 40, 435 - 480 and 650 - 774 residues), HNH domain (520 - 628 residues), WED domain (788 - 909 residues) and PI domain (910 - 1053 residues). The PI domain is divided into topoisomerase homology (TOPO) domain and C-terminal domain (CTD). The RuvC domain is constituted of 3 separate motifs (RuvC-I - III) and is associated with the HNH domain and PI domain. The HNH domain is linked to RuvC-II and RuvC-III via L1 (481 - 519 residues) linker and L2 (629 - 649 residues) linker, respectively. The WED domain and RucV domain are linked by "phosphate lock" loop (775 - 787 residues) (H. Nishimasu et al., Cell, Volume 162, Issue 5, pp. 1113-1126).

In one embodiment of the present invention, the continuous deletion region present between the 481-position and the 649-position in the amino acid sequence shown in SEQ ID NO: 2 is
(i) entire L1 domain (481- to 519-positions),
(ii) entire HNH domain (520- to 628-positions), and
(iii) entire L2 domain (629- to 649-positions)

### (embodiment 1-1).

In one embodiment of the present invention, the continuous deletion region present between the 481-position and the 649-position in the amino acid sequence shown in SEQ ID NO: 2 is
(i) a part of L1 domain (482- to 519-positions),
(ii) entire HNH domain (520- to 628-positions), and
(iii) a part of L2 domain (629- to 647-positions)

### (embodiment 1-2).

In one embodiment of the present invention, the continuous deletion region present between the 481-position and the 649-position in the amino acid sequence shown in SEQ ID NO: 2 is
(i) a part of L1 domain (482- to 519-positions), and
(ii) entire HNH domain (520- to 628)

### (embodiment 1-3).

In another embodiment of the present invention, the present invention provides a protein (embodiment 2) having binding ability to guide RNA and further having mutations at the 45-position and/or the 163-position in addition to the mutations in the aforementioned embodiments 1, 1-1, 1-2 and 1-3 .

The mutation(s) at the 45-position and/or the 163-position are/is specifically substitution of glutamic acid with basic amino acid, preferably with lysine, arginine or histidine, more preferably with lysine.

In another embodiment of the present invention, the present invention provides a protein (embodiment 3) consisting of a sequence comprising an amino acid sequence resulting from substitution of glutamic acid at the 45-position and/or the 163-position with other amino acid in the amino acid sequence shown in SEQ ID NO: 2, and having a binding ability to guide RNA.

The mutation(s) at the 45-position and/or the 163-position are/is specifically substitution of glutamic acid with basic amino acid, preferably with lysine, arginine or histidine, more preferably with lysine.

As a method for optionally creating "a continuous deletion region between the 481-position and the 649-position" in the amino acid sequence shown in SEQ ID NO: 2, and a method for substituting "glutamic acid at the 45-position and/or the 163-position with other amino acid" in the amino acid sequence shown in SEQ ID NO: 2, a method including introducing a conventional site-specific mutation into a DNA encoding a predetermined amino acid sequence, and then expressing the DNA by a conventional method can be mentioned. Examples of the method for introducing a site-specific mutation include a method using amber mutation (gapped-duplex method, Nucleic Acids Res., 12, 9441-9456 (1984)), a method by PCR using a primer for mutagenesis, and the like. In addition, it can be easily performed according to the manual and using the Q5 Site-Directed Mutagenesis Kit (NEB).

In another embodiment of the present invention, the present invention provides a protein (embodiment 4) that is functionally equivalent to the proteins of the aforementioned embodiments 1, 1-1, 1-2, 1-3, 2 and 3. To be functionally equivalent to the proteins of the aforementioned embodiments 1, 1-1, 1-2, 1-3, 2 and 3, the amino acid sequence having identity of 80% or more at a site other than the positions where the mutations have been applied in the SEQ ID NO: 2 in the aforementioned embodiments 1, 1-1, 1-2, 1-3, 2 and 3 and needs to have a binding ability to guide RNA. When amino acids are increased or decreased due to mutation, the "site other than the position(s) where the mutation(s) has(have) been applied" can be interpreted to mean a "site other than the position(s) corresponding to the position(s) where the mutation(s) has (have) been applied". This identity is preferably 80% or more, more preferably 85% or more, even more preferably 90% or more, particularly preferably 95% or more, and most preferably 99% or more. The amino acid sequence identity can be determined by a method known per se. For example, amino acid sequence identity (%) can be determined using a program conventionally used in the pertinent field (e.g., BLAST, FASTA, etc.) by default. In another aspect, identity (%) is determined by any algorithm known in the pertinent field, such as algorithms of Needleman et al. (1970) (J. Mol. Biol. 48: 444-453), Myers and Miller (CABIOS, 1988, 4: 11-17) and the like. The algorithm of Needleman et al. is incorporated into the GAP program in the GCG software package (available at www.gcg.com) and the identity (%) can be determined using, for example, any of BLOSUM 62 matrix and PAM250 matrix, as well as gap weight: 16, 14, 12, 10, 8, 6 or 4, and length weight: 1, 2, 3, 4, 5 or 6. The algorithm of Myers and Miller is incorporated into the ALIGN program that is a part of the GCG sequence alignment software package. When the ALIGN program is used to compare amino acid sequences, for example, PAM120 weight residue table, gap length penalty 12, and gap penalty 4 can be used.

As a protein functionally equivalent to the proteins of the aforementioned embodiments 1, 1-1, 1-2, 1-3, 2 and 3, a protein (embodiment 4-1) which comprises one to several amino acids substituted, deleted, inserted and/or added at site(s) other than the positions where the mutations have been applied in the SEQ ID NO: 2 in the aforementioned embodiment 1, 1-1, 1-2, 1-3, 2 and 3 and having the binding ability to guide RNA is provided. When amino acids are increased or decreased due to mutation, the "site other than the position(s) where the mutation(s) has(have) been applied" can be interpreted to mean a "site other than the position(s) corresponding to the position(s) where the mutation(s) has(have) been applied".

As a technique for artificially performing "substitution, deletion, insertion and/or addition of amino acid", for example, a method including applying conventional site specific mutation introduction to DNA encoding a predetermined amino acid sequence, and thereafter expressing the DNA by a conventional method can be mentioned. Examples of the site specific mutation introduction method include a method using amber mutation (gapped duplex method, Nucleic Acids Res., 12, 9441-9456 (1984)), a PCR method using a mutation introduction primer and the like. In addition, it can be easily performed according to the manual and using the Q5 Site-Directed Mutagenesis Kit (NEB).

The number of the amino acids modified above is at least one residue, specifically one or several, or more than that. Among the aforementioned substitution, deletion, insertion and addition, substitution of amino acid is particularly preferred. The substitution is more preferably substitution with an amino acid having similar properties such as hydrophobicity, charge, pK, and characteristic of steric structure and the like. Examples of the substitution include substitution within the groups of i) glycine, alanine; ii) valine, isoleucine, leucine; iii) aspartic acid, glutamic acid, asparagine, glutamine; iv) serine, threonine; v) lysine, arginine; vi) phenylalanine, tyrosine.

In the dSaCas9 variant of the present invention, the dSaCas9 variant protein is in a state of being cleaved by the deletion mutation, and the both ends of the deletion region are linked by a linker. That is, in the dSaCas9 variant of the present invention, amino acids each adjacent to the deletion region are linked by a linker consisting of 3 to 10 amino acid residues. Due to the linkage, the dSaCas9 variant of the present invention has a continuous amino acid sequence.

The linker (hereinafter to be also referred to as the linker of the present invention) is not particularly limited as long as it can link both ends of a cleaved protein and does not influence the function thereof. Preferably, it is a group capable of adopting a intrinsically disordered structure that binds to other protein while freely changing its own shape according to the protein, and preferably a linker composed of 3 to 10 amino acid residues which is constituted of glycine (G) and serine (S). More preferably, the linker of the present invention is a peptide residue having a length of 5 - 9 amino acids. Specifically, the following residues can be mentioned.
- SGGGS- (SEQ ID NO: 3)
- GGSGGS- (SEQ ID NO: 4)
- SGSGSGSG- (SEQ ID NO: 5)
- SGSGSGSGS- (SEQ ID NO: 6)

The introduction of linker in each variant can also be performed by a method including performing conventional site-specific mutagenesis on the DNA encoding a predetermined amino acid sequence to insert a base sequence encoding the linker, and thereafter expressing the DNA by a conventional method. Examples of the method for site-specific mutagenesis include methods same as those described above.

The dSaCas9 variant in the present embodiment can be produced according to, for example, the method indicated below. First, a host is transformed using a vector containing a nucleic acid that encodes the dSaCas9 variant of the present invention. Then, the host is cultured to express the aforementioned protein. Conditions such as medium composition, culture temperature, duration of culturing or addition of inducing agents can be determined by a person with ordinary skill in the art in accordance with known methods so that the transformant grows and the aforementioned protein is efficiently produced. In addition, in the case of having incorporated a selection marker in the form of an antibiotic resistance gene in an expression vector, the transformant can be selected by adding antibiotic to the medium. Then, dSaCas9 variant of the present invention is obtained by purifying the aforementioned protein expressed by the host according to a method known per se.

There are no particular limitations on the host, and examples thereof include animal cells, plant cells, insect cells and microorganisms such as Escherichia coli, Bacillus subtilis or yeast. Preferred host is an animal cell.

### <dSaCas9 variant-guide RNA complex>

In one embodiment thereof, the present invention provides a protein-RNA complex provided with the protein indicated in the previous section on <dSaCas9 variant> and guide RNA containing a polynucleotide composed of a base sequence complementary to a base sequence located 1 to 20 to 24 bases upstream from a proto-spacer adjacent motif (PAM) sequence in a target double-stranded polynucleotide.

The aforementioned protein and the aforementioned guide RNA are able to form a protein-RNA complex by mixing in vitro and in vivo under mild conditions. Mild conditions refer to a temperature and pH of a degree that does not cause protein decomposition or denaturation, and the temperature is preferably 4°C to 40°C, while the pH is preferably 4 to 10.

In addition, the duration of mixing and incubating the aforementioned protein and the aforementioned guide RNA is preferably 0.5 hr to 1 hr. The complex formed by the aforementioned protein and the aforementioned guide RNA is stable and is able to maintain stability even if allowed to stand for several hours at room temperature.

### <CRISPR-Cas Vector System>

In one embodiment thereof, the present invention provides a CRISPR-Cas vector system provided with a first vector containing a gene encoding a protein indicated in the previous section on <dSaCas9 variant>, and a second vector containing a guide RNA containing a polynucleotide composed of a base sequence complementary to a base sequence located 1 to 20 to 24 bases upstream from PAM sequence in a target double-stranded polynucleotide.

In another embodiment, the present invention provides a CRISPR-Cas vector system in which a gene encoding a protein indicated in the previous section on <dSaCas9 variant>, and a guide RNA containing a polynucleotide composed of a base sequence complementary to a base sequence located 1 to 20 to 24 bases upstream from PAM sequence in a target double-stranded polynucleotide are contained in the same vector.

The guide RNA is suitably designed to contain in the 5'-terminal region thereof a polynucleotide composed of a base sequence complementary to a base sequence located from 1 to 20 to 24 bases, and preferably to 22 to 24 bases, upstream from a PAM sequence in a target double-stranded polynucleotide. Moreover, the guide RNA may also contain one or more polynucleotides composed of a base sequence allowing the obtaining of a hairpin structure composed of base sequences non-complementary to a target double-stranded polynucleotide symmetrically arranged so as to form a complementary sequence having a single point as the axis thereof.

The vector of the present embodiment is preferably an expression vector. Examples of the expression vector that can be used include E. coli-derived plasmids such as pBR322, pBR325, pUC12 or pUC13; B. subtilis-derived plasmids such as pUB110, pTP5 or pC194; yeast-derived plasmids such as pSH19 or pSH15; bacteriophages such as λphages; viruses such as adenovirus, adeno-associated virus, lentivirus, vaccinia virus, baculovirus or cytomegalovirus; and modified vectors thereof. In view of the activation of gene expression in vivo, a virus vector, particularly an adeno-associated virus, is preferable.

In the aforementioned expression vector, there are no particular limitations on the promoters for expression of the aforementioned dSaCas9 variant protein or the aforementioned guide RNA, and examples thereof that can be used include promoters for expression in animal cells such as EF1α promoter, SRα promoter, SV40 promoter, LTR promoter, cytomegalovirus (CMV) promoter or HSV-tk promoter, promoters for expression in plant cells such as the 35S promoter of cauliflower mosaic virus (CaMV) or rubber elongation factor (REF) promoter, and promoters for expression in insect cells such as polyhedrin promoter or p10 promoter. These promoters can be suitably selected according to the aforementioned dSaCas9 variant protein and the aforementioned guide RNA, or the type of cells expressing the aforementioned Cas9 protein and the aforementioned guide RNA.

The aforementioned expression vector may also further have a multi-cloning site, enhancer, splicing signal, polyadenylation signal, selection marker (drug resistant) and promoter thereof, or replication origin and the like.

### <Method for Site-Specifically Modifying Target Double-Stranded Polynucleotide>

### [First Embodiment]

In one embodiment thereof, the present invention provides a method for site-specifically modifying a target double-stranded polynucleotide, provided with:
a step for mixing and incubating a target double-stranded polynucleotide, a protein and a guide RNA, and
a step for having the aforementioned protein modify the aforementioned target double-stranded polynucleotide at a binding site located upstream of a PAM sequence; wherein,
the aforementioned target double-stranded polynucleotide has a PAM sequence,
the aforementioned protein is a protein shown in the aforementioned <dSaCas9 variant>, and
the aforementioned guide RNA contains a polynucleotide composed of a base sequence complementary to a base sequence located 1 to 20 to 24 bases upstream from the aforementioned PAM sequence in the aforementioned target double-stranded polynucleotide.

In the present embodiment, the target double-stranded polynucleotide is not particular limited as long as it has a PAM sequence.

In the present embodiment, the protein and guide RNA are as described in the aforementioned <dSaCas9 variant>.

The following provides a detailed explanation of the method for site-specifically modifying a target double-stranded polynucleotide.

First, the aforementioned protein and the aforementioned guide RNA are mixed and incubated under mild conditions. Mild conditions are as previously described. The incubation time is preferably 0.5 hr to 1 hr. A complex formed by the aforementioned protein and the aforementioned guide RNA is stable and is able to maintain stability even if allowed to stand for several hours at room temperature.

Next, the aforementioned protein and the aforementioned guide RNA form a complex on the aforementioned target double-stranded polynucleotide. The aforementioned protein recognizes PAM sequences, and binds to the aforementioned target double-stranded polynucleotide at a binding site located upstream of the PAM sequence. Successively, a target double-stranded polynucleotide modified to meet the purpose can be obtained in a region determined by the complementary binding of the aforementioned guide RNA and the aforementioned double-stranded polynucleotide.

In the present description, the "modification" means that the target double-stranded polynucleotide changes structurally or functionally. For example, structural or functional change of the target double-stranded polynucleotide by the addition of a functional protein or a base sequence can be mentioned. By the modification, the function of the target double-stranded polynucleotide can be altered, deleted, enhanced, or suppressed, and a new function can be added.

Since the dSaCas9 variant of the present invention does not have endonuclease activity, the protein can bind to the aforementioned target double-stranded polynucleotide at the binding site located upstream of the PAM sequence bond, but stays there and cannot cleave the polynucleotide. Therefore, for example, when a labeled protein such as fluorescent protein (e.g., GFP) and the like is fused to the protein, the labeled protein can be bound to the target double-stranded polynucleotide via dSaCas9 variant protein-guide RNA. By appropriately selecting a substance to be bound to the dSaCas9 variant, various functions can be imparted to the target double-stranded polynucleotide.

Furthermore, transcriptional regulator protein or domain can be linked to the N-terminal or C-terminal of the dSaCas9 variant protein. Examples of the transcriptional regulator or domain thereof include transcriptional activator or domain thereof (e.g., VP64, VP160, NF-κB p65), transcriptional silencer or domain thereof (e.g., hetero chromatin protein 1(HP1)), and transcriptional inhibitor or domain thereof (e.g., Kruppel-associated box (KRAB), ERF repressor domain (ERD), mSin3A interaction domain (SID)).

It is also possible to link an enzyme that modifies the methylation state of DNA (e.g., DNA methyltransferase (DNMT), TET)), or an enzyme that modifies a histone subunit (e.g., histone acetyltransferase (HAT), histone deacetylase (HDAC), histone methyltransferase, histone demethylase).

### [Second Embodiment]

In the present embodiment, an expression step may be further provided prior to the incubation step in which the protein described in the previous section on <dSaCas9 variant> and guide RNA are expressed using the previously described CRISPR-Cas vector system.

In the expression step of the present embodiment, dSaCas9 variant protein and guide RNA are first expressed using the aforementioned CRISPR-Cas vector system. A specific expression method includes transforming a host using an expression vector containing a gene that encodes dSaCas9 variant protein and an expression vector containing guide RNA, respectively (or expression vector simultaneously containing gene encoding dSaCas9 variant protein and guide RNA). Then, the host is cultured to express the dSaCas9 variant protein and guide RNA. Conditions such as medium composition, culture temperature, duration of culturing or addition of inducing agents can be determined by a person with ordinary skill in the art in accordance with known methods so that the transformant grows and the aforementioned protein is efficiently produced. In addition, in the case of having incorporated a selection marker in the form of an antibiotic resistance gene in the expression vector, the transformant can be selected by adding antibiotic to the medium. Then, the dSaCas9 variant protein and guide RNA are obtained by purifying the dSaCas9 variant protein and guide RNA expressed by the host according to a suitable method.

### <Method for Site-Specifically Modifying Target Double-Stranded Polynucleotide in Cells>

In one embodiment thereof, the present invention provides a method for site-specifically modifying a target double-stranded polynucleotide in cells, provided with:
a step for introducing the previously described CRISPR-Cas vector system into a cell and expressing protein described in the previous section on <dSaCas9 variant> and guide RNA,
a step for having the aforementioned protein bind with the aforementioned target double-stranded polynucleotide at a binding site located upstream of a PAM sequence, and
a step for obtaining a modified target double-stranded polynucleotide in a region determined by complementary binding between the aforementioned guide RNA and the aforementioned target double-stranded polynucleotide; wherein
the aforementioned guide RNA contains a polynucleotide composed of a base sequence complementary to a base sequence located 1 to 20 to 24 bases upstream from the aforementioned PAM sequence in the aforementioned target double-stranded polynucleotide.

In the expression step of the present embodiment, first, dSaCas9 variant protein and guide RNA are expressed in a cell using the aforementioned CRISPR-Cas vector system.

Examples of organisms serving as the origin of the cells targeted for application of the method of the present embodiment include prokaryote, yeast, animal, plant, insect and the like. There are no particular limitations on the aforementioned animals, and examples thereof include, but are not limited to, human, monkey, dog, cat, rabbit, swine, bovine, mouse, rat and the like. In addition, the type of organism serving as the source of the cells can be arbitrarily selected according to the desired type or objective of the target double-stranded polynucleotide.

Examples of animal-derived cells targeted for application of the method of the present embodiment include, but are not limited to, germ cells (such as sperm or ova), somatic cells composing the body, stem cells, progenitor cells, cancer cells isolated from the body, cells isolated from the body that are stably maintained outside the body as a result of having become immortalized (cell line), and cells isolated from the body for which the nuclei have been artificially replaced.

Examples of somatic cells composing the body include, but are not limited to, cells harvested from arbitrary tissue such as the skin, kidneys, spleen, adrenals, liver, lungs, ovaries, pancreas, uterus, stomach, small intestine, large intestine, urinary bladder, prostate gland, testes, thymus, muscle, connective tissue, bone, cartilage, vascular tissue, blood, heart, eyes, brain or neural tissue. Specific examples of somatic cells include, but are not limited to, fibroblasts, bone marrow cells, immune cells (e.g., B lymphocytes, T lymphocytes, neutrophils, macrophages or monocytes etc.), erythrocytes, platelets, osteocytes, bone marrow cells, pericytes, dendritic cells, keratinocytes, adipocytes, mesenchymal cells, epithelial cells, epidermal cells, endothelial cells, intravascular endothelial cells, lymphatic endothelial cells, hepatocytes, pancreatic islet cells (e.g., α cells, β cells, δ cells, ε cells or PP cells etc.), chondrocytes, cumulus cells, glia cells, nerve cells (neurons), oligodendrocytes, microglia cells, astrocytes, cardiomyocytes, esophageal cells, muscle cells (e.g., smooth muscle cells or skeletal muscle cells etc.), melanocytes and mononuclear cells, and the like.

Stem cells refer to cells having both the ability to self-replicate as well as the ability to differentiate into a plurality of other cell lines. Examples of stem cells include, but are not limited to, embryonic stem cells (ES cells), embryonic tumor cells, embryonic germ stem cells, induced pluripotent stem cells (iPS cells), neural stem cells, hematopoietic stem cells, mesenchymal stem cells, hepatic stem cells, pancreatic stem cells, muscle stem cells, germ stem cells, intestinal stem cells, cancer stem cells and hair follicle stem cells, and the like.

Cancer cells are cells derived from somatic cells that have acquired reproductive integrity. Examples of the origins of cancer cells include, but are not limited to, breast cancer (e.g., invasive ductal carcinoma, non-invasive ductal carcinoma, inflammatory breast cancer etc.), prostate cancer (e.g., hormone-dependent prostate cancer or hormone-independent prostate cancer etc.), pancreatic cancer (e.g., pancreatic ductal carcinoma etc.), gastric cancer (e.g., papillary adenocarcinoma, mucinous carcinoma, adenosquamous carcinoma etc.), lung cancer (e.g., non-small cell lung cancer, small cell lung cancer, malignant mesothelioma etc.), colon cancer (e.g., gastrointestinal stromal tumor etc.), rectal cancer (e.g., gastrointestinal stromal tumor etc.), colorectal cancer (e.g., familial colorectal cancer, hereditary non-polyposis colon cancer, gastrointestinal stromal tumor etc.), small intestine cancer (e.g., non-Hodgkin's lymphoma, gastrointestinal stromal tumor etc.), esophageal cancer, duodenal cancer, tongue cancer, pharyngeal cancer (e.g., nasopharyngeal carcinoma, oropharyngeal carcinoma, hypopharyngeal carcinoma etc.), head and neck cancer, salivary gland cancer, brain tumor (e.g., pineal astrocytoma, pilocytic astrocytoma, diffuse astrocytoma, anaplastic astrocytoma etc.), schwannoma, liver cancer (e.g., primary liver cancer, extrahepatic bile duct cancer etc.), kidney cancer (e.g., renal cell carcinoma, transitional cell carcinoma of the renal pelvis and ureter etc.), gallbladder cancer, bile duct cancer, pancreatic cancer, endometrial carcinoma, cervical cancer, ovarian cancer (e.g., epithelial ovarian cancer, extragonadal germ cell tumor, ovarian germ cell tumor, ovarian low malignant potential tumor etc.), bladder cancer, urethral cancer, skin cancer (e.g., intraocular (ocular) melanoma, Merkel cell carcinoma etc.), hemangioma, malignant lymphoma (e.g., reticulum cell sarcoma, lymphosarcoma, Hodgkin's etc.), melanoma (malignant melanoma), thyroid cancer (e.g., medullary thyroid cancer etc.), parathyroid cancer, nasal cancer, paranasal cancer, bone tumor (e.g., osteosarcoma, Ewing's tumor, uterine sarcoma, soft tissue sarcoma etc.), metastatic medulloblastoma, angiofibroma, protuberant dermatofibrosarcoma, retinal sarcoma, penile cancer, testicular tumor, pediatric solid tumor (e.g., Wilms tumor or pediatric kidney tumor etc.), Kaposi's sarcoma, AIDS-induced Kaposi's sarcoma, maxillary sinus tumor, fibrous histiocytoma, leiomyosarcoma, rhabdomyosarcoma, chronic myeloproliferative disease and leukemia (e.g., acute myelogenous leukemia, acute lymphoblastic leukemia etc.).

Cell lines refer to cells that have acquired reproductive integrity through artificial manipulation ex vivo. Examples of cell lines include, but are not limited to, HCT116, Huh7, HEK293 (human embryonic kidney cells), HeLa (human cervical cancer cell line), HepG2 (human liver cancer cell line), UT7/TPO (human leukemia cell line), CHO (Chinese hamster ovary cell line), MDCK, MDBK, BHK, C-33A, HT-29, AE-1, 3D9, NsO/1, Jurkat, NIH3T3, PC12, S2, Sf9, Sf21, High Five and Vero.

Introduction of the CRISPR-Cas vector system into cells can be carried out using a method suitable for the viable cells used, and examples thereof include electroporation method, heat shock method, calcium phosphate method, lipofection method, DEAE dextran method, microinjection method, particle gun method, methods using viruses, and methods using commercially available transfection reagents such as FuGENE (registered trade mark) 6 Transfection Reagent (manufactured by Roche), Lipofectamine 2000 Reagent (manufactured by Invitrogen Corp.), Lipofectamine LTX Reagent (manufactured by Invitrogen Corp.) or Lipofectamine 3000 Reagent (manufactured by Invitrogen Corp.).

The subsequent modification step is the same as the method shown in the aforementioned <Method for Site-Specifically Modifying Target Double-Stranded nucleotide>

### [First Embodiment].

By the modification of the target double-stranded polynucleotide in this embodiment, cells with modified target double-stranded polynucleotide can be obtained.

While the present invention is explained in more detail in the following by referring to Examples, they do not limit the scope of the present invention.

### [Example]

### Example 1: Evaluation of DNA binding affinity of dSaCas9 variant

### (Method)

### 1. Cloning

Using NEB Q5 Site-Directed Mutagenesis Kit, a predetermined deletion region was made in dSaCas9 gene, a gene encoding a linker was introduced, and a KRAB gene as a transcriptional regulator was fused to produce various dSaCas9 variants (Fig. 1). The expression suppression activity of these variants was examined using MYD88 gene. All gene constructs of dSaCas9 variant were incorporated into pX601 vectors (F. Ann Ran et al., Nature 2015; 520(7546); pp.186-191). For the DNA binding assay, crRNA sequence; GGAGCCACAGTTCTTCCACGG (SEQ ID NO: 7) was fused with tracrRNA sequence; GTTTTAGTACTCTGGAAACAGAATCTACTAAAACAAGGCAAAATGCCGTGTTTATCACGTCAA CTTGTTGGCGAGATTTTTTT (SEQ ID NO: 8) to form a guide RNA (sgRNA) to be expressed from the vector.

As the guide RNA (control sgRNA) sequence of the control, the following sequence was used: ACGGAGGCTAAGCGTCGCAA (SEQ ID NO: 9).

### 2. Cell transfection

HEK293FT cells were seeded in a 24-well plate at a density of 75,000 cells per well 24 hr before transfection and cultured in DMEM medium supplemented with 10% FBS, 2 mM fresh L-glutamine, 1 mM sodium pyruvate and non-essential amino acid. The cells were transfected according to the manual and using 500 ng of each dSaCas9 variant (repressor) expression vector, each sgRNA expression vector, and 1.5 µl of Lipofectamine 2000 (Life technologies). For the gene expression analysis, the cells were recovered at 48-72 hr after transfection, dissolved in RLT buffer (Qiagen), and the total RNA was extracted using RNeasy kit (Qiagen).

### 3. Gene expression analysis

For the Taqman analysis, cDNA was prepared from 1.5 µg of the total RNA by using 20 µl volume TaqMan™ High-Capacity RNA-to-cDNA Kit (Applied Biosystems). The prepared cDNA was diluted 20-fold and 6.33 µl was used for each Taqman reaction. The Taqman primers and probes for the MYD88 gene were obtained from Applied Biosystems. In Roche LightCycler 96 or LightCycler 480, the Taqman reaction was run using Taqman gene expression master mix (ThermoFisher), reaction was run using Taqman gene expression master mix (ThermoFisher), and the analysis was performed using LightCycler 96 analysis software.

Taqman probe product IDs:
MYD88: Hs01573837_g1 (FAM)
HPRT: Hs99999909_m1 (FAM, VIC)

Taqman QPCR condition:
Step 1; 95C 10 min
Step 2; 95C 15 sec
Step 3; 60C 30 sec
Repeat Step 2 and 3; 40 times

### (Results)

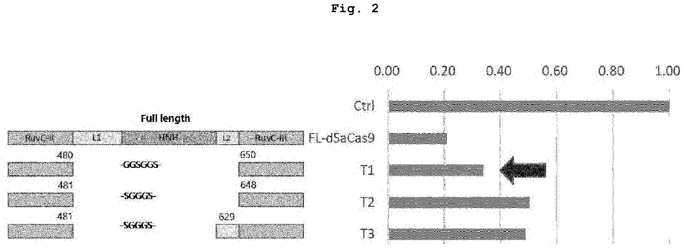

When compared with the control, the gene expression level in the dSaCas9 variant of the present invention was as low as that of the wild-type dSaCas9 (Fig. 2). From the results, it was shown that the binding ability to the guide RNA, and further, the DNA binding affinity, were maintained even though the dSaCas9 variant of the present invention has a deletion region and is reduced to a size of about 80% that of the full-length dSaCas9.

Various point mutations were further introduced into T1 which showed particularly high DNA binding affinity in the above-mentioned results, and the effects thereof were confirmed. The results are shown in Fig. 3.

It was confirmed that M12 (T1 variant in which glutamic acid at the 45-position was substituted with lysine) had DNA binding affinity superior to that of T1.

Various point mutations were further introduced into M12, and the effects thereof were confirmed. The results are shown in Fig. 4.

It was confirmed that M15 (M12 variant in which glutamic acid at the 163-position was substituted with lysine) had DNA binding affinity superior to that of M12.

### [Industrial Applicability]

According to the present invention, a dSaCas9 protein that is miniaturized while maintaining a DNA binding ability can be obtained. Use of the miniaturized dSaCas9 protein makes it possible to mount many genes into vectors, and thus provides various genome editing techniques.

This application is based on a provisional patent application No. 62/682,244 filed in the US (filing date: June 8, 2018), the contents of which are incorporated in full herein.

## Claims

1. A protein having a binding ability to guide RNA and consisting of a sequence comprising an amino acid sequence wherein a continuous deletion region is present between the 481-position and the 649-position in the amino acid sequence shown in SEQ ID NO: 2, the deletion region comprising
(i) all or a part of L1 domain (481- to 519-positions), and
(ii) entire HNH domain (520- to 628-positions), and further optionally comprising
(iii) all or a part of L2 domain (629- to 649-positions), wherein amino acids adjacent to each of the deletion region are linked by a linker consisting of 3 to 10 amino acid residues.

2. The protein according to claim 1, wherein the deletion region comprises
(i) entire L1 domain (481- to 519-positions),
(ii) entire HNH domain region (520- to 628-positions), and
(iii) entire L2 domain (629- to 649-positions).

3. The protein according to claim 1, wherein the deletion region comprises
(i) a part of L1 domain (482- to 519-positions),
(ii) entire HNH domain (520- to 628-positions), and
(iii) a part of L2 domain (629- to 647-positions).

4. The protein according to claim 1, wherein the deletion region comprises
(i) a part of L1 domain (482- to 519-positions), and
(ii) entire HNH domain (520- to 628-positions).

5. A protein consisting of a sequence comprising an amino acid sequence resulting from substitution of glutamic acid (E) at the 45-position and/or the 163-position with other amino acid in the amino acid sequence shown in SEQ ID NO: 2, and having a binding ability to guide RNA.

6. The protein according to claim 5, wherein said other amino acid is a basic amino acid.

7. The protein according to claim 6, wherein the basic amino acid is lysine (K).

8. The protein according to any one of claims 1 to 4, wherein glutamic acid (E) at the 45-position and/or the 163-position are/is substituted with other amino acid(s).

9. The protein according to claim 8, wherein said other amino acid is a basic amino acid.

10. The protein according to claim 9, wherein the basic amino acid is lysine (K).

11. The protein according to any one of claims 1 to 8, wherein the linker is a 5 - 9 amino acid length linker composed of glycine (G) and serine (S).

12. The protein according to any one of claims 1 to 11, wherein the linker is selected from the following:
- SGGGS-
- GGSGGS-
- SGSGSGSG-
- SGSGSGSGS-.

13. The protein according to any one of claims 1 to 12, having identity of 80% or more at a site other than the mutated and/or deleted positions in the SEQ ID NO: 2.

14. The protein according to any one of claims 1 to 12, wherein one to several amino acids are substituted, deleted, inserted and/or added at a site other than the mutated and/or deleted positions in the SEQ ID NO: 2.

15. The protein according to any one of claims 1 to 14, wherein a transcriptional regulator protein or domain is linked.

16. The protein according to claim 15, wherein the transcriptional regulator is a transcriptional activator.

17. The protein according to claim 15, wherein the transcriptional regulator is a transcriptional silencer or a transcriptional inhibitor.

18. A nucleic acid encoding the protein according to any one of claims 1 to 17.

19. A protein-RNA complex provided with the protein according to any one of claims 1 to 18 and a guide RNA comprising a polynucleotide composed of a base sequence complementary to a base sequence located 1 to 20 to 24 bases upstream from a proto-spacer adjacent motif (PAM) sequence in a target double-stranded polynucleotide.

20. A method for site-specifically modifying a target double-stranded polynucleotide, including
a step of mixing and incubating a target double-stranded polynucleotide, a protein and a guide RNA, and
a step of having the protein modify the target double-stranded polynucleotide at a binding site located upstream of a PAM sequence; wherein,
the protein is the protein according to any one of claims 1 to 17, and
the guide RNA contains a polynucleotide composed of a base sequence complementary to a base sequence located 1 to 20 to 24 bases upstream from the PAM sequence in the target double-stranded polynucleotide.

21. A method for increasing expression of a target gene in a cell, comprising expressing the protein according to claim 16 and one or plural guide RNAs for the target gene in the cell.

22. A method for decreasing expression of a target gene in a cell, comprising expressing the protein according to claim 17 and one or plural guide RNAs for the target gene in the cell.

23. The method according to claim 21 or 22, wherein the cell is a eukaryotic cell.

24. The method according to claim 21 or 22, wherein the cell is a yeast cell, a plant cell or an animal cell.


SEQUENCE LISTING

<110>  EdiGENE Corporation

<120>  Modified Cas9 protein and use thereof

<130>  092916

<150>  US62/682,244
<151>  2018-6-8

<160>  11

<170>  PatentIn version 3.5

<210>  1
<211>  3162
<212>  DNA
<213>  Staphylococcus aureus

<220>
<221>  CDS
<222>  (1)..(3162)

<220>
<221>  gene
<222>  (1)..(3162)
<223>  dSaCas9

<400>  2
atg aag cgg aac tac atc ctg ggc ctg gcc atc ggc atc acc agc gtg          48
Met Lys Arg Asn Tyr Ile Leu Gly Leu Ala Ile Gly Ile Thr Ser Val
1               5                   10                  15

ggc tac ggc atc atc gac tac gag aca cgg gac gtg atc gat gcc ggc          96
Gly Tyr Gly Ile Ile Asp Tyr Glu Thr Arg Asp Val Ile Asp Ala Gly
            20                  25                  30

gtg cgg ctg ttc aaa gag gcc aac gtg gaa aac aac gag ggc agg cgg          144
Val Arg Leu Phe Lys Glu Ala Asn Val Glu Asn Asn Glu Gly Arg Arg
        35                  40                  45

agc aag aga ggc gcc aga agg ctg aag cgg cgg agg cgg cat aga atc          192
Ser Lys Arg Gly Ala Arg Arg Leu Lys Arg Arg Arg Arg His Arg Ile
    50                  55                  60

cag aga gtg aag aag ctg ctg ttc gac tac aac ctg ctg acc gac cac          240
Gln Arg Val Lys Lys Leu Leu Phe Asp Tyr Asn Leu Leu Thr Asp His
65                  70                  75                  80

agc gag ctg agc ggc atc aac ccc tac gag gcc aga gtg aag ggc ctg          288
Ser Glu Leu Ser Gly Ile Asn Pro Tyr Glu Ala Arg Val Lys Gly Leu
                85                  90                  95

agc cag aag ctg agc gag gaa gag ttc tct gcc gcc ctg ctg cac ctg          336
Ser Gln Lys Leu Ser Glu Glu Glu Phe Ser Ala Ala Leu Leu His Leu
            100                 105                 110

gcc aag aga aga ggc gtg cac aac gtg aac gag gtg gaa gag gac acc          384
Ala Lys Arg Arg Gly Val His Asn Val Asn Glu Val Glu Glu Asp Thr
        115                 120                 125

ggc aac gag ctg tcc acc aaa gag cag atc agc cgg aac agc aag gcc 432
Gly Asn Glu Leu Ser Thr Lys Glu Gln Ile Ser Arg Asn Ser Lys Ala
    130                 135                 140

ctg gaa gag aaa tac gtg gcc gaa ctg cag ctg gaa cgg ctg aag aaa 480
Leu Glu Glu Lys Tyr Val Ala Glu Leu Gln Leu Glu Arg Leu Lys Lys
145                 150                 155                 160

gac ggc gaa gtg cgg ggc agc atc aac aga ttc aag acc agc gac tac 528
Asp Gly Glu Val Arg Gly Ser Ile Asn Arg Phe Lys Thr Ser Asp Tyr
                165                 170                 175

gtg aaa gaa gcc aaa cag ctg ctg aag gtg cag aag gcc tac cac cag 576
Val Lys Glu Ala Lys Gln Leu Leu Lys Val Gln Lys Ala Tyr His Gln
            180                 185                 190

ctg gac cag agc ttc atc gac acc tac atc gac ctg ctg gaa acc cgg 624
Leu Asp Gln Ser Phe Ile Asp Thr Tyr Ile Asp Leu Leu Glu Thr Arg
        195                 200                 205

cgg acc tac tat gag gga cct ggc gag ggc agc ccc ttc ggc tgg aag 672
Arg Thr Tyr Tyr Glu Gly Pro Gly Glu Gly Ser Pro Phe Gly Trp Lys
    210                 215                 220

gac atc aaa gaa tgg tac gag atg ctg atg ggc cac tgc acc tac ttc 720
Asp Ile Lys Glu Trp Tyr Glu Met Leu Met Gly His Cys Thr Tyr Phe
225                 230                 235                 240

ccc gag gaa ctg cgg agc gtg aag tac gcc tac aac gcc gac ctg tac 768
Pro Glu Glu Leu Arg Ser Val Lys Tyr Ala Tyr Asn Ala Asp Leu Tyr
                245                 250                 255

aac gcc ctg aac gac ctg aac aat ctc gtg atc acc agg gac gag aac 816
Asn Ala Leu Asn Asp Leu Asn Asn Leu Val Ile Thr Arg Asp Glu Asn
            260                 265                 270

gag aag ctg gaa tat tac gag aag ttc cag atc atc gag aac gtg ttc 864
Glu Lys Leu Glu Tyr Tyr Glu Lys Phe Gln Ile Ile Glu Asn Val Phe
        275                 280                 285

aag cag aag aag aag ccc acc ctg aag cag atc gcc aaa gaa atc ctc 912
Lys Gln Lys Lys Lys Pro Thr Leu Lys Gln Ile Ala Lys Glu Ile Leu
    290                 295                 300

gtg aac gaa gag gat att aag ggc tac aga gtg acc agc acc ggc aag 960
Val Asn Glu Glu Asp Ile Lys Gly Tyr Arg Val Thr Ser Thr Gly Lys
305                 310                 315                 320

ccc gag ttc acc aac ctg aag gtg tac cac gac atc aag gac att acc 1008
Pro Glu Phe Thr Asn Leu Lys Val Tyr His Asp Ile Lys Asp Ile Thr
                325                 330                 335

gcc cgg aaa gag att att gag aac gcc gag ctg ctg gat cag att gcc 1056
Ala Arg Lys Glu Ile Ile Glu Asn Ala Glu Leu Leu Asp Gln Ile Ala
            340                 345                 350

aag atc ctg acc atc tac cag agc agc gag gac atc cag gaa gaa ctg 1104
Lys Ile Leu Thr Ile Tyr Gln Ser Ser Glu Asp Ile Gln Glu Glu Leu
        355                 360                 365

acc aat ctg aac tcc gag ctg acc cag gaa gag atc gag cag atc tct 1152
Thr Asn Leu Asn Ser Glu Leu Thr Gln Glu Glu Ile Glu Gln Ile Ser
    370                 375                 380

aat ctg aag ggc tat acc ggc acc cac aac ctg agc ctg aag gcc atc 1200
Asn Leu Lys Gly Tyr Thr Gly Thr His Asn Leu Ser Leu Lys Ala Ile
385                 390                 395                 400

aac ctg atc ctg gac gag ctg tgg cac acc aac gac aac cag atc gct 1248
Asn Leu Ile Leu Asp Glu Leu Trp His Thr Asn Asp Asn Gln Ile Ala
                405                 410                 415

atc ttc aac cgg ctg aag ctg gtg ccc aag aag gtg gac ctg tcc cag 1296
Ile Phe Asn Arg Leu Lys Leu Val Pro Lys Lys Val Asp Leu Ser Gln
            420                 425                 430

cag aaa gag atc ccc acc acc ctg gtg gac gac ttc atc ctg agc ccc 1344
Gln Lys Glu Ile Pro Thr Thr Leu Val Asp Asp Phe Ile Leu Ser Pro
        435                 440                 445

gtc gtg aag aga agc ttc atc cag agc atc aaa gtg atc aac gcc atc 1392
Val Val Lys Arg Ser Phe Ile Gln Ser Ile Lys Val Ile Asn Ala Ile
    450                 455                 460

atc aag aag tac ggc ctg ccc aac gac atc att atc gag ctg gcc cgc 1440
Ile Lys Lys Tyr Gly Leu Pro Asn Asp Ile Ile Ile Glu Leu Ala Arg
465                 470                 475                 480

gag aag aac tcc aag gac gcc cag aaa atg atc aac gag atg cag aag 1488
Glu Lys Asn Ser Lys Asp Ala Gln Lys Met Ile Asn Glu Met Gln Lys
                485                 490                 495

cgg aac cgg cag acc aac gag cgg atc gag gaa atc atc cgg acc acc 1536
Arg Asn Arg Gln Thr Asn Glu Arg Ile Glu Glu Ile Ile Arg Thr Thr
            500                 505                 510

ggc aaa gag aac gcc aag tac ctg atc gag aag atc aag ctg cac gac 1584
Gly Lys Glu Asn Ala Lys Tyr Leu Ile Glu Lys Ile Lys Leu His Asp
        515                 520                 525

atg cag gaa ggc aag tgc ctg tac agc ctg gaa gcc atc cct ctg gaa 1632
Met Gln Glu Gly Lys Cys Leu Tyr Ser Leu Glu Ala Ile Pro Leu Glu
    530                 535                 540

gat ctg ctg aac aac ccc ttc aac tat gag gtg gac cac atc atc ccc 1680
Asp Leu Leu Asn Asn Pro Phe Asn Tyr Glu Val Asp His Ile Ile Pro
545                 550                 555                 560

aga agc gtg tcc ttc gac aac agc ttc aac aac aag gtg ctc gtg aag 1728
Arg Ser Val Ser Phe Asp Asn Ser Phe Asn Asn Lys Val Leu Val Lys
                565                 570                 575

cag gaa gaa gcc agc aag aag ggc aac cgg acc cca ttc cag tac ctg 1776
Gln Glu Glu Ala Ser Lys Lys Gly Asn Arg Thr Pro Phe Gln Tyr Leu
            580                 585                 590

agc agc agc gac agc aag atc agc tac gaa acc ttc aag aag cac atc 1824
Ser Ser Ser Asp Ser Lys Ile Ser Tyr Glu Thr Phe Lys Lys His Ile
        595                 600                 605

ctg aat ctg gcc aag ggc aag ggc aga atc agc aag acc aag aaa gag 1872
Leu Asn Leu Ala Lys Gly Lys Gly Arg Ile Ser Lys Thr Lys Lys Glu
    610                 615                 620

tat ctg ctg gaa gaa cgg gac atc aac agg ttc tcc gtg cag aaa gac 1920
Tyr Leu Leu Glu Glu Arg Asp Ile Asn Arg Phe Ser Val Gln Lys Asp

625                 630                 635                 640

ttc atc aac cgg aac ctg gtg gat acc aga tac gcc acc aga ggc ctg       1968
Phe Ile Asn Arg Asn Leu Val Asp Thr Arg Tyr Ala Thr Arg Gly Leu
                645                 650                 655

atg aac ctg ctg cgg agc tac ttc aga gtg aac aac ctg gac gtg aaa       2016
Met Asn Leu Leu Arg Ser Tyr Phe Arg Val Asn Asn Leu Asp Val Lys
            660                 665                 670

gtg aag tcc atc aat ggc ggc ttc acc agc ttt ctg cgg cgg aag tgg       2064
Val Lys Ser Ile Asn Gly Gly Phe Thr Ser Phe Leu Arg Arg Lys Trp
        675                 680                 685

aag ttt aag aaa gag cgg aac aag ggg tac aag cac cac gcc gag gac       2112
Lys Phe Lys Lys Glu Arg Asn Lys Gly Tyr Lys His His Ala Glu Asp
    690                 695                 700

gcc ctg atc att gcc aac gcc gat ttc atc ttc aaa gag tgg aag aaa       2160
Ala Leu Ile Ile Ala Asn Ala Asp Phe Ile Phe Lys Glu Trp Lys Lys
705                 710                 715                 720

ctg gac aag gcc aaa aaa gtg atg gaa aac cag atg ttc gag gaa aag       2208
Leu Asp Lys Ala Lys Lys Val Met Glu Asn Gln Met Phe Glu Glu Lys
                725                 730                 735

cag gcc gag agc atg ccc gag atc gaa acc gag cag gag tac aaa gag       2256
Gln Ala Glu Ser Met Pro Glu Ile Glu Thr Glu Gln Glu Tyr Lys Glu
            740                 745                 750

atc ttc atc acc ccc cac cag atc aag cac att aag gac ttc aag gac       2304
Ile Phe Ile Thr Pro His Gln Ile Lys His Ile Lys Asp Phe Lys Asp
        755                 760                 765

tac aag tac agc cac cgg gtg gac aag aag cct aat aga gag ctg att       2352
Tyr Lys Tyr Ser His Arg Val Asp Lys Lys Pro Asn Arg Glu Leu Ile
    770                 775                 780

aac gac acc ctg tac tcc acc cgg aag gac gac aag ggc aac acc ctg       2400
Asn Asp Thr Leu Tyr Ser Thr Arg Lys Asp Asp Lys Gly Asn Thr Leu
785                 790                 795                 800

atc gtg aac aat ctg aac ggc ctg tac gac aag gac aat gac aag ctg       2448
Ile Val Asn Asn Leu Asn Gly Leu Tyr Asp Lys Asp Asn Asp Lys Leu
                805                 810                 815

aaa aag ctg atc aac aag agc ccc gaa aag ctg ctg atg tac cac cac       2496
Lys Lys Leu Ile Asn Lys Ser Pro Glu Lys Leu Leu Met Tyr His His
            820                 825                 830

gac ccc cag acc tac cag aaa ctg aag ctg att atg gaa cag tac ggc       2544
Asp Pro Gln Thr Tyr Gln Lys Leu Lys Leu Ile Met Glu Gln Tyr Gly
        835                 840                 845

gac gag aag aat ccc ctg tac aag tac tac gag gaa acc ggg aac tac       2592
Asp Glu Lys Asn Pro Leu Tyr Lys Tyr Tyr Glu Glu Thr Gly Asn Tyr
    850                 855                 860

ctg acc aag tac tcc aaa aag gac aac ggc ccc gtg atc aag aag att       2640
Leu Thr Lys Tyr Ser Lys Lys Asp Asn Gly Pro Val Ile Lys Lys Ile
865                 870                 875                 880

aag tat tac ggc aac aaa ctg aac gcc cat ctg gac atc acc gac gac       2688

Lys Tyr Tyr Gly Asn Lys Leu Asn Ala His Leu Asp Ile Thr Asp Asp
                885                 890                 895

tac ccc aac agc aga aac aag gtc gtg aag ctg tcc ctg aag ccc tac      2736
Tyr Pro Asn Ser Arg Asn Lys Val Val Lys Leu Ser Leu Lys Pro Tyr
            900                 905                 910

aga ttc gac gtg tac ctg gac aat ggc gtg tac aag ttc gtg acc gtg      2784
Arg Phe Asp Val Tyr Leu Asp Asn Gly Val Tyr Lys Phe Val Thr Val
        915                 920                 925

aag aat ctg gat gtg atc aaa aaa gaa aac tac tac gaa gtg aat agc      2832
Lys Asn Leu Asp Val Ile Lys Lys Glu Asn Tyr Tyr Glu Val Asn Ser
    930                 935                 940

aag tgc tat gag gaa gct aag aag ctg aag aag atc agc aac cag gcc      2880
Lys Cys Tyr Glu Glu Ala Lys Lys Leu Lys Lys Ile Ser Asn Gln Ala
945                 950                 955                 960

gag ttt atc gcc tcc ttc tac aac aac gat ctg atc aag atc aac ggc      2928
Glu Phe Ile Ala Ser Phe Tyr Asn Asn Asp Leu Ile Lys Ile Asn Gly
                965                 970                 975

gag ctg tat aga gtg atc ggc gtg aac aac gac ctg ctg aac cgg atc      2976
Glu Leu Tyr Arg Val Ile Gly Val Asn Asn Asp Leu Leu Asn Arg Ile
            980                 985                 990

gaa gtg aac atg atc gac atc acc  tac cgc gag tac ctg  gaa aac atg      3024
Glu Val Asn Met Ile Asp Ile Thr  Tyr Arg Glu Tyr Leu  Glu Asn Met
        995                 1000                 1005

aac gac  aag agg ccc ccc agg  atc att aag aca atc  gcc tcc aag      3069
Asn Asp  Lys Arg Pro Pro Arg  Ile Ile Lys Thr Ile  Ala Ser Lys
    1010                 1015                 1020

acc cag  agc att aag aag tac  agc aca gac att ctg  ggc aac ctg      3114
Thr Gln  Ser Ile Lys Lys Tyr  Ser Thr Asp Ile Leu  Gly Asn Leu
    1025                 1030                 1035

tat gaa  gtg aaa tct aag aag  cac cct cag atc atc  aaa aag ggc      3159
Tyr Glu  Val Lys Ser Lys Lys  His Pro Gln Ile Ile  Lys Lys Gly
    1040                 1045                 1050

taa                                                                  3162

<210>  2
<211>  1053
<212>  PRT
<213>  Staphylococcus aureus

<400>  2

Met Lys Arg Asn Tyr Ile Leu Gly Leu Ala Ile Gly Ile Thr Ser Val
1               5                   10                  15

Gly Tyr Gly Ile Ile Asp Tyr Glu Thr Arg Asp Val Ile Asp Ala Gly
            20                  25                  30

Val Arg Leu Phe Lys Glu Ala Asn Val Glu Asn Asn Glu Gly Arg Arg
        35                  40                  45

Ser Lys Arg Gly Ala Arg Arg Leu Lys Arg Arg Arg Arg His Arg Ile
    50                  55                  60

Gln Arg Val Lys Lys Leu Leu Phe Asp Tyr Asn Leu Leu Thr Asp His
65                  70                  75                  80

Ser Glu Leu Ser Gly Ile Asn Pro Tyr Glu Ala Arg Val Lys Gly Leu
                85                  90                  95

Ser Gln Lys Leu Ser Glu Glu Glu Phe Ser Ala Ala Leu Leu His Leu
            100                 105                 110

Ala Lys Arg Arg Gly Val His Asn Val Asn Glu Val Glu Glu Asp Thr
        115                 120                 125

Gly Asn Glu Leu Ser Thr Lys Glu Gln Ile Ser Arg Asn Ser Lys Ala
    130                 135                 140

Leu Glu Glu Lys Tyr Val Ala Glu Leu Gln Leu Glu Arg Leu Lys Lys
145                 150                 155                 160

Asp Gly Glu Val Arg Gly Ser Ile Asn Arg Phe Lys Thr Ser Asp Tyr
                165                 170                 175

Val Lys Glu Ala Lys Gln Leu Leu Lys Val Gln Lys Ala Tyr His Gln
            180                 185                 190

Leu Asp Gln Ser Phe Ile Asp Thr Tyr Ile Asp Leu Leu Glu Thr Arg
        195                 200                 205

Arg Thr Tyr Tyr Glu Gly Pro Gly Glu Gly Ser Pro Phe Gly Trp Lys
    210                 215                 220

Asp Ile Lys Glu Trp Tyr Glu Met Leu Met Gly His Cys Thr Tyr Phe
225                 230                 235                 240

Pro Glu Glu Leu Arg Ser Val Lys Tyr Ala Tyr Asn Ala Asp Leu Tyr
                245                 250                 255

Asn Ala Leu Asn Asp Leu Asn Asn Leu Val Ile Thr Arg Asp Glu Asn
            260                 265                 270

Glu Lys Leu Glu Tyr Tyr Glu Lys Phe Gln Ile Ile Glu Asn Val Phe
        275                 280                 285

Lys Gln Lys Lys Lys Pro Thr Leu Lys Gln Ile Ala Lys Glu Ile Leu

290                 295                 300

Val Asn Glu Glu Asp Ile Lys Gly Tyr Arg Val Thr Ser Thr Gly Lys
305                 310                 315                 320

Pro Glu Phe Thr Asn Leu Lys Val Tyr His Asp Ile Lys Asp Ile Thr
                325                 330                 335

Ala Arg Lys Glu Ile Ile Glu Asn Ala Glu Leu Leu Asp Gln Ile Ala
            340                 345                 350

Lys Ile Leu Thr Ile Tyr Gln Ser Ser Glu Asp Ile Gln Glu Glu Leu
        355                 360                 365

Thr Asn Leu Asn Ser Glu Leu Thr Gln Glu Glu Ile Glu Gln Ile Ser
    370                 375                 380

Asn Leu Lys Gly Tyr Thr Gly Thr His Asn Leu Ser Leu Lys Ala Ile
385                 390                 395                 400

Asn Leu Ile Leu Asp Glu Leu Trp His Thr Asn Asp Asn Gln Ile Ala
                405                 410                 415

Ile Phe Asn Arg Leu Lys Leu Val Pro Lys Lys Val Asp Leu Ser Gln
            420                 425                 430

Gln Lys Glu Ile Pro Thr Thr Leu Val Asp Asp Phe Ile Leu Ser Pro
        435                 440                 445

Val Val Lys Arg Ser Phe Ile Gln Ser Ile Lys Val Ile Asn Ala Ile
    450                 455                 460

Ile Lys Lys Tyr Gly Leu Pro Asn Asp Ile Ile Ile Glu Leu Ala Arg
465                 470                 475                 480

Glu Lys Asn Ser Lys Asp Ala Gln Lys Met Ile Asn Glu Met Gln Lys
                485                 490                 495

Arg Asn Arg Gln Thr Asn Glu Arg Ile Glu Glu Ile Ile Arg Thr Thr
            500                 505                 510

Gly Lys Glu Asn Ala Lys Tyr Leu Ile Glu Lys Ile Lys Leu His Asp
        515                 520                 525

Met Gln Glu Gly Lys Cys Leu Tyr Ser Leu Glu Ala Ile Pro Leu Glu
    530                 535                 540

Asp Leu Leu Asn Asn Pro Phe Asn Tyr Glu Val Asp His Ile Ile Pro
545                 550                 555                 560

Arg Ser Val Ser Phe Asp Asn Ser Phe Asn Asn Lys Val Leu Val Lys
                565                 570                 575

Gln Glu Glu Ala Ser Lys Lys Gly Asn Arg Thr Pro Phe Gln Tyr Leu
            580                 585                 590

Ser Ser Ser Asp Ser Lys Ile Ser Tyr Glu Thr Phe Lys Lys His Ile
        595                 600                 605

Leu Asn Leu Ala Lys Gly Lys Gly Arg Ile Ser Lys Thr Lys Lys Glu
    610                 615                 620

Tyr Leu Leu Glu Glu Arg Asp Ile Asn Arg Phe Ser Val Gln Lys Asp
625                 630                 635                 640

Phe Ile Asn Arg Asn Leu Val Asp Thr Arg Tyr Ala Thr Arg Gly Leu
                645                 650                 655

Met Asn Leu Leu Arg Ser Tyr Phe Arg Val Asn Asn Leu Asp Val Lys
            660                 665                 670

Val Lys Ser Ile Asn Gly Gly Phe Thr Ser Phe Leu Arg Arg Lys Trp
        675                 680                 685

Lys Phe Lys Lys Glu Arg Asn Lys Gly Tyr Lys His His Ala Glu Asp
    690                 695                 700

Ala Leu Ile Ile Ala Asn Ala Asp Phe Ile Phe Lys Glu Trp Lys Lys
705                 710                 715                 720

Leu Asp Lys Ala Lys Lys Val Met Glu Asn Gln Met Phe Glu Glu Lys
                725                 730                 735

Gln Ala Glu Ser Met Pro Glu Ile Glu Thr Glu Gln Glu Tyr Lys Glu
            740                 745                 750

Ile Phe Ile Thr Pro His Gln Ile Lys His Ile Lys Asp Phe Lys Asp
        755                 760                 765

Tyr Lys Tyr Ser His Arg Val Asp Lys Lys Pro Asn Arg Glu Leu Ile
    770                 775                 780

Asn Asp Thr Leu Tyr Ser Thr Arg Lys Asp Asp Lys Gly Asn Thr Leu
785                 790                 795                 800

Ile Val Asn Asn Leu Asn Gly Leu Tyr Asp Lys Asp Asn Asp Lys Leu
                805                 810                 815

Lys Lys Leu Ile Asn Lys Ser Pro Glu Lys Leu Leu Met Tyr His His
            820                 825                 830

Asp Pro Gln Thr Tyr Gln Lys Leu Lys Leu Ile Met Glu Gln Tyr Gly
        835                 840                 845

Asp Glu Lys Asn Pro Leu Tyr Lys Tyr Tyr Glu Glu Thr Gly Asn Tyr
    850                 855                 860

Leu Thr Lys Tyr Ser Lys Lys Asp Asn Gly Pro Val Ile Lys Lys Ile
865                 870                 875                 880

Lys Tyr Tyr Gly Asn Lys Leu Asn Ala His Leu Asp Ile Thr Asp Asp
                885                 890                 895

Tyr Pro Asn Ser Arg Asn Lys Val Val Lys Leu Ser Leu Lys Pro Tyr
            900                 905                 910

Arg Phe Asp Val Tyr Leu Asp Asn Gly Val Tyr Lys Phe Val Thr Val
        915                 920                 925

Lys Asn Leu Asp Val Ile Lys Lys Glu Asn Tyr Tyr Glu Val Asn Ser
    930                 935                 940

Lys Cys Tyr Glu Glu Ala Lys Lys Leu Lys Lys Ile Ser Asn Gln Ala
945                 950                 955                 960

Glu Phe Ile Ala Ser Phe Tyr Asn Asn Asp Leu Ile Lys Ile Asn Gly
                965                 970                 975

Glu Leu Tyr Arg Val Ile Gly Val Asn Asn Asp Leu Leu Asn Arg Ile
            980                 985                 990

Glu Val Asn Met Ile Asp Ile Thr  Tyr Arg Glu Tyr Leu  Glu Asn Met
        995                 1000                1005

Asn Asp  Lys Arg Pro Pro Arg  Ile Ile Lys Thr Ile  Ala Ser Lys
    1010                1015                1020

Thr Gln  Ser Ile Lys Lys Tyr  Ser Thr Asp Ile Leu  Gly Asn Leu
    1025                1030                1035

Tyr Glu  Val Lys Ser Lys Lys  His Pro Gln Ile Ile  Lys Lys Gly
    1040                1045                1050

<210>  3
<211>  5
<212>  PRT
<213>  Artificial Sequence

<220>
<223>  linker

<400>  3

Ser Gly Gly Gly Ser
1               5

<210>  4
<211>  6
<212>  PRT
<213>  Artificial Sequence

<220>
<223>  linker

<400>  4

Gly Gly Ser Gly Gly Ser
1               5

<210>  5
<211>  8
<212>  PRT
<213>  Artificial Sequence

<220>
<223>  linker

<400>  5

Ser Gly Ser Gly Ser Gly Ser Gly
1               5

<210>  6
<211>  9
<212>  PRT
<213>  Artificial Sequence

<220>
<223>  linker

<400>  6

Ser Gly Ser Gly Ser Gly Ser Gly Ser
1               5

<210>  7
<211>  21
<212>  RNA
<213>  Artificial Sequence

<220>

<223>  Synthetic sequence for crRNA

<400>  7
ggagccacag uucuuccacg g                                                21


<210>  8
<211>  83
<212>  RNA
<213>  Artificial Sequence

<220>
<223>  Synthetic sequence for tracRNA

<400>  8
guuuuaguac ucuggaaaca gaaucuacua aaacaaggca aaaugccgug uuuaucacgu      60

caacuuguug gcgagauuuu uuu                                              83


<210>  9
<211>  20
<212>  RNA
<213>  Artificial Sequence

<220>
<223>  Synthetic sequence for control sgRNA

<400>  9
acggaggcua agcgucgcaa                                                  20


<210>  10
<211>  8
<212>  PRT
<213>  Artificial Sequence

<220>
<223>  linker

<400>  10

Gly Gly Gly Ser Gly Gly Gly Gly
1               5


<210>  11
<211>  7
<212>  PRT
<213>  Artificial Sequence

<220>
<223>  linker

<400>  11

Lys Arg Arg Arg Arg His Arg
1               5